(19) Europäisches Patentamt
European Patent Office
Office européen des brevets

(11) Publication number: **0 151 319**
**B1**

(12) # EUROPEAN PATENT SPECIFICATION

(45) Date of publication of patent specification: **20.04.88**

(51) Int. Cl.$^4$: **C 07 D 221/14**

(21) Application number: **84201377.3**

(22) Date of filing: **23.03.82**

(60) Publication number of the earlier application in accordance with Art. 76 EPC: **0 061 341**

(54) **Hexahydrobenzoderquinoline derivatives and their preparation.**

(30) Priority: **24.03.81 US 247047**

(43) Date of publication of application: **14.08.85 Bulletin 85/33**

(45) Publication of the grant of the patent: **20.04.88 Bulletin 88/16**

(84) Designated Contracting States: **AT BE CH DE FR GB IT LI LU NL SE**

(56) References cited:

**HELVETICA CHIMICA ACTA, vol. 56, fasc. 2, no. 73, 1973, pages 759-773, Basel, CH; F. SCHNEIDER et al.: "Chemische und pharmakologische Studien über Derivate des Benzoderchinolins(I)**

(73) Proprietor: **ELI LILLY AND COMPANY
307, East McCarty Street
Indianapolis Indiana 46285 (US)**

(72) Inventor: **Kornfeld, Edmund Carl
5159 East 76th Court
Indianapolis Indiana 46250 (US)**
Inventor: **Bach, Nicholas James
4269 Burkhart Street East Drive
Indianapolis Indiana (US)**
Inventor: **Titus, Robert Daniel
1203 North Emerson
Indianapolis Indiana 46219 (US)**

(74) Representative: **Tapping, Kenneth George et al
Lilly Industries Limited Patent Department
Erl Wood Manor
Windlesham Surrey, GU20 6PH (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 151 319 B1

**Description**

Our co-pending application No. 82301483.2, publication No. 61341 concerns 6-substituted hexahydroindazolo or hexahydroisoindolo isoquinolines of the formula

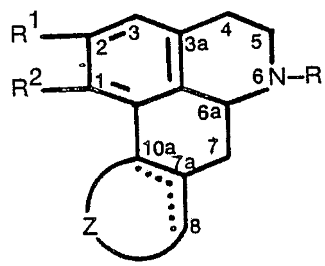

I

wherein

R is H, $(C_1—C_3)$ alkyl, allyl or benzyl;

$R^1$ and $R^2$ are independently H, OH or $(C_1—C_3)$-alkoxy, with the limitation that when either $R^1$ or $R^2$ is OH, then the other $R^1$ or $R^2$ can not be $(C_1—C_3)$alkoxy;

Z is

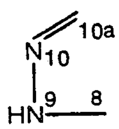 , 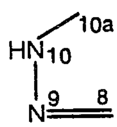 , or 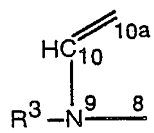 ;

$R^3$ is H or $-\overset{\overset{\displaystyle O}{\|}}{C}-(C_1-C_2)$alkyl;

the dotted line represents the presence of one double bond at either 7a—10a or 7a—8; and the acid addition salts thereof.

The compounds of formula I above are prepared by reacting an 8-dimethylaminoethylene derivative of the formula

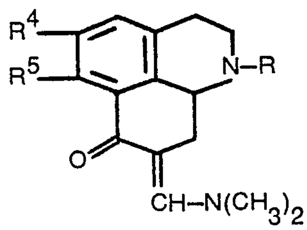

II

wherein

R is defined as above;

$R^4$ and $R^5$ are independently H or $(C_1—C_3)$alkoxy;

with

$$R^6—NH_2$$

where $R^6$ is $NH_2—$, in a non-reactive solvent, when Z is

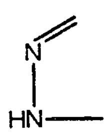 or 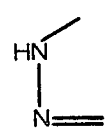 ; or

$R^6$ is $MO-\overset{\overset{\displaystyle O}{\|}}{C}-CH_2-$,

where M is K or Na, and a $(C_1—C_2)$alkyl anhydride, when Z is

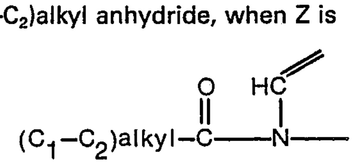 ;

optionally followed by hydrolysis when $R^3$ is

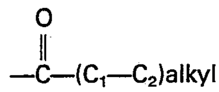

to obtain the compounds of formula I where $R^3$ is H;

optionally folowed by dealkylating the compounds where $R^1$ and/or $R^2$ are $(C_1—C_3)$alkoxy to obtain the compounds of formula I where $R^1$ and/or $R^2$ are OH; and

optionally followed by reductive alkylation when R is H to obtain the compounds where R is $(C_1—C_3)$alkyl or allyl.

The compounds of formula I above, when Z is

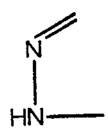 or 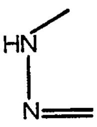 ;

result in the formation of 6-substituted hexahydroindazolo isoquinoline products. These products are prepared by reacting a compound of formula II above with anhydrous hydrazine in a non-reactive solvent. Examples of some suitable solvents are $C_1—C_6$ alcohols, *i.e.* methanol, ethanol, chloroform, methylenedichloride, ethers, especially diethyl ether, benzene and toluene. The temperature range to run this reaction is from about 0—100°C, with room temperature being preferred.

The compounds of formula I above, when Z is

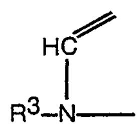

result in the formation of 6-substituted hexahydroisoindolo isoquinoline products. These products are prepared by reacting a compound of formula II above with an alkali metal glycinate, e.g. K or Na glycinate, followed by treatment with a $(C_1—C_2)$alkylanhydride, e.g. acetic anhydride. The preferred temperature is reflux. The preferred solvents are $(C_1—C_3)$alcohols. This reaction forms a product of formula I where $R^3$ is

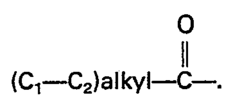

To obtain the products of formula I where $R^3$ is H, the

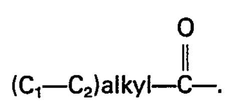

group is hydrolyzed, e.g. with a strong base, such as sodium ethoxide, potassium hydroxide, sodium hydroxide, sodium methoxide, and potassium t-butoxide, all in an alcohol solvent.

This invention relates to novel intermediates useful in the preparation of compounds of formula I, which are:

8-dimethylaminomethylene compounds of the formula

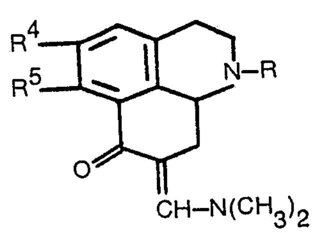

II

wherein

R is H, benzyl, $(C_1—C_3)$alkyl or allyl; and

$R^4$ and $R^5$ are independently H or $(C_1—C_3)$- alkoxy; and 7-oxo-hexahydrobenzo[de]quinolines of the formula

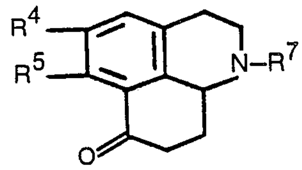

IV

wherein

$R^7$ is ($C_1$—$C_3$)alkyl, allyl, or benzyl;

$R^4$ and $R^5$ are independently ($C_1$—$C_3$)alkoxy, or H, with the limitation that then $R^4$ and $R^5$ are both ($C_1$—$C_3$)alkoxy, then $R^7$ can not be methyl.

The compounds of formula II above are prepared by reacting a suitably 1-substituted 7-oxo-2,3,7,8,9,9a-hexahydro-1H-benzo[de]quinoline of the formula

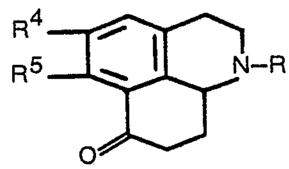

III

wherein:

$R^4$, $R^5$ and R are defined as above, with dimethylformamide acetal, tris(dimethylamino)methane, or bis(dimethylamino)methoxymethane.

The compounds of formula IV above are prepared by reacting a compound of the formula

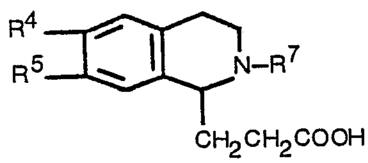

V

wherein

$R^4$, $R^5$ and $R^7$ are defined as above, with a strong acid cyclizing agent.

The starting 1-unsubstituted-7-oxo-benzo[de]quinolines, formula III, are prepared by somewhat different routes depending on whether $R^4$ is H or alkoxy. For example, Schneider et al. *Helv. Chim. Acta. 56,* 759 (1973) have prepared 5,6-dimethoxy-7-oxo-2,3,7,8,9,9a-hexahydro-1H-benzo[de]quinoline (formula III above where $R^4$ and $R^5$ are methoxy and R is methyl) by cyclizing N-(3,4-dimethoxyphenylethyl) β-(methoxycarbonyl)propionamide to yield methyl β-(6,7-dimethoxy-3,4-dihydro-1-isoquinolinyl)propionate. Quaternization with RX wherein X is a halogen and R has its previous meaning followed by borohydride reduction of the quaternary salt and hydrolysis of the reduction product gives β-(6,7-dimethoxy-1,2,3,4-tetrahydro-1-isoquinolyl)propionic acid, cyclization of which with pyrophosphoric acid, oleum or the like yields the desired 7-oxo derivative.

Where there is no activation of the benzene ring ortho to a side chain as in a 3,4-dimethoxy ethylamine, as in the Schneider et al. procedure, a somewhat different synthetic route is used. In this procedure, 1-methylisoquinoline is condensed with chloral to yield, after purification, 1-(3,3,3-trichloro-2-hydroxypropyl)isoquinoline of the formula

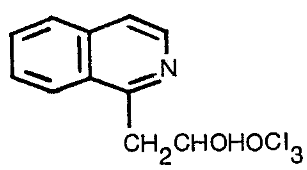

VI

Treatment of VI with base, as for example, an aqueous alkali metal hydroxide, serves both to hydrolyze the trichloromethyl group to a carboxylic acid and to dehydrate the resulting molecule to yield a β-(1-isoquinolinyl)acrylic acid of the formula

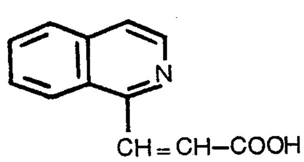

VII

Next, the acrylic acid group of VII is esterified via the acid chloride as with a lower alkanol. The resulting acrylate ester group is then hydrogenated, to a propionate group (formula VIII) preferably with Raney nickel although other catalysts such as Adams catalyst ($PtO_2$) may also be used. Moderate hydrogen pressures (3,515—4,218 g.cm$^2$(3.4—4.1 × $10^5$Pa)) are employed and the acrylate ester diluted with a lower alkanol, preferably the same alkanol used to esterify the acrylic acid.

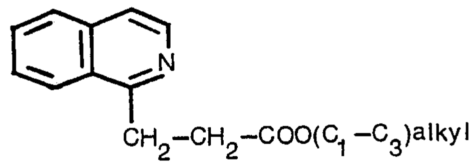

VIII

Next, the ring nitrogen is quaternized, as with methyl, ethyl, n-propyl or allyl iodide, or benzyl bromide or methylfluorosulfonate and the quaternary salt reduced with a metal hydride reducing agent which does not also reduce the ester group. Ordinarily, sodium borohydride is used. The product of this series of reactions is a β-(1,2,3,4-tetrahydro-1-isoquinolinyl)propionate (IX), wherein in $R^7$ is ($C_1$—$C_3$)alkyl, allyl or benzyl

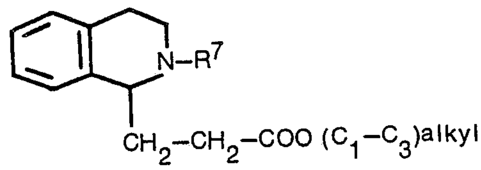

IX

Hydrolysis of the propionate group to the free acid yields a β-[1-methyl, (ethyl, propyl, allyl, benzyl) 1,2,3,4-tetrahydro-1-isoquinolinyl)propionic acid (Va) usually obtained as an acid addition salt such as a hydrochloride salt.

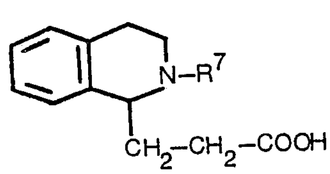

Va

A cyclization procedure, using oleum, $P_2O_5$ plus methanesulfonic acid, polyphosphoric acid or the like as the strong acid cyclizing agent, yields the desired intermediate 1-methyl(ethyl, propyl, allyl, benzyl)-7-oxo-2,3,7,8,9,9a-hexahydro-1H-benzo[de]quinoline (IV) (III when $R^4$ and $R^5$ are H and $R^7$ is $C_1$—$C_3$alkyl, allyl or benzyl).

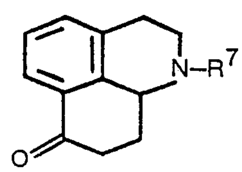

IV

When $R^7$ is methyl, the above two steps in the synthetic procedure can be reversed, i.e., the β-(1-isoquinolinyl)acrylic acid VII can be esterified and the acrylate ester quaternized as with methylfluorosulfonate and the methyl quaternary salt reduced with borohydride to yield an acrylate ester of structure.

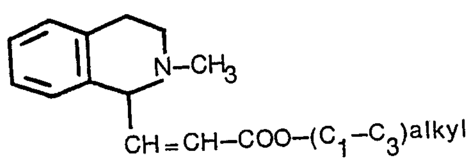

X

Hydrogenation of the acrylate double bond over Raney nickel followed by hydrolysis of the resulting propionate ester yields a propionic acid derivative (Va wherein $R^7$ is $CH_3$). Cyclization of this derivative yields a tricyclic ketone (IV wherein $R^7$ is $CH_3$) produced by an alternate pathway.

If quaternization in either procedure is carried out with a benzyl halide (or pseudo halide), the final product of the reaction sequence will be a 6-benzyl-4,5,6,6a,7,9-hexahydroindazolo[7,6,5-ij]isoquinoline and its 4,5,6,6a,7,10-hexahydro tautomer. The benzyl group can then be removed by catalytic hydrogenation according to standard procedures to produce those intermediates of this invention of formula I in which R

is H, which intermediates can be reductively alkylated, for example with formaldehyde, acetaldehyde, propionaldehyde or acrylaldehyde and sodium cyanoborohydride in a lower alkanol such as methanol to produce the corresponding N-methyl, N-ethyl, N-n-propyl or N-allyl compounds of this invention (I in which R = ($C_1$—$C_3$)alkyl or allyl). Catalytic hydrogenation can also be used.

In formula II, when $R^4$ and $R^5$ are both ($C_1$—$C_3$)alkoxy or one is ($C_1$—$C_3$)alkoxy and the other H, the alkoxy group can be dealkylated by well known methods, e.g. 48% HBr in glacial acetic acid, to yield the corresponding OH group for $R^1$ and/or $R^2$ in formula I.

Compounds according to formula I as well as the 3-ring intermediates II, III, and IV contain an asymmetric center-carbon 7a in formula I and carbon 9a in formulas II, III or IV. These compounds represented by the above formulas exist in two diastereoisomeric forms. In the ordinary synthetic procedure, the two stereoisomers are produced in equal amounts and the product is isolated as a dl pair or racemate. The racemate can be separated into its component stereoisomers by methods available in the art, or by salt formation with an optically active acid. It is not known whether each stereoisomer will have some degree of physiologic activity or whether all such activity will reside in a single isomer. This invention covers isomers possessing dopaminergic or dopamine antagonist activity or both. Racemic mixtures of a physiologically active isomer with an inactive isomer are useful in providing the pharmacologic affect of the active isomer without the time and expense of an isomer separation.

This invention is further illustrated by the following specific examples. The nmr data was run on a 100 mHz machine in $CDCl_3$.

## Example 1

Preparation of 6-Methyl-4,5,6,6a,7,9-hexahydroindazolo[7,6,5-ij]isoquinoline and 6-Methyl-4,5,6,6a,7,10-hexahydroindazolo[7,6,5-ij]isoquinoline

A reaction mixture was prepared by dissolving 50 g. of 1-methylisoquinoline, 40 g. pyridine and 70 g. of chloral in 400 ml. of dioxane. The reaction mixture was heated to reflux temperature under a nitrogen atmosphere for 2.75 hours and was then allowed to remain overnight at ambient temperature. Evaporation of the volatile constituents left a residue which was dissolved in ether. The ethereal solution was filtered through Florisil® and the filtrate saturated with gaseous hydrogen chloride. 1-(3,3,3-trichloro-2-hydroxypropyl)isoquinoline hydrochloride, being insoluble in ether, precipitated and the precipitate was collected by filtration. Recrystallization of the filter cake from methanol-ether yielded crystals melting with decomposition at about 217°C.

| | | | | |
|---|---|---|---|---|
| Analysis Calculated: | C, 44.07; | H, 3.39; | N, 4.28; | Cl, 43.36 |
| Found: | C, 44.31; | H, 3.12; | N, 4.02; | Cl, 43.38 |

In a second run based on 15 g. of starting 1-methylisoquinoline, there were obtained 29.28 g. of 1-(3,3,3-trichloro-2-hydroxypropyl)isoquinoline hydrochloride melting with decomposition at about 210°C.

Next, a solution was prepared containing 100 ml. of 50% aqueous sodium hydroxide, 50 ml. of water and 150 ml. of ethanol. To this solution was added 10 g. of 1-(3,3,3-trichloro-2-hydroxypropyl)isoquinoline hydrochloride. The reaction mixture was kept at about 50°C. during the addition. After the addition had been completed, the reaction mixture was stirred at room temperature under a nitrogen atmosphere overnight. The reaction mixture was then cooled and made acid by the addition of 12N aqueous hydrochloric acid to a pH of about 6. The resulting solution was extracted several times with a mixture of chloroform and isopropanol. The organic extracts were separated and combined and dried. Evaporation of the dried solution *in vacuo* yielded a solid residue comprising β-(1-isoquinolinyl)acrylic acid formed in the above reaction. Recrystallization of the residue from a mixture of methanol and chloroform yielded crystals melting at about 172—174°C. with vigorous decomposition.

| | | | |
|---|---|---|---|
| Analysis Calculated: | C, 72.35; | H, 4.55; | N, 7.03 |
| Found: | C, 72.14; | H, 4.56; | N, 6.71 |

In a second run using 10 g. of 1-(3,3,3-trichloro-2-hydroxypropyl)isoquinline hydrochloride, about 3.5 g. of the acrylic acid were obtained.

Three and one-half grams of β-(1-isoquinolinyl)acrylic acid were dissolved in about 250 ml. of methanol and the resulting solution cooled in an ice-water mixture. 2 ml. of thionyl chloride were added in dropwise fashion. The reaction mixture was stirred with cooling for about an hour and then at room temperature for about four days. The volatile constituents were removed by evaporation *in vacuo* and the reaction mixture diluted with aqueous sodium bicarbonate. The aqueous layer was extracted with chloroform, the chloroform layer separated and washed with saturated aqueous sodium chloride and then dried. Evaporation of the chloroform yielded methyl β-(1-isoquinolinyl)acrylate formed in the above reaction. The residue was suspended in ether and the resulting suspension chromatographed over 150 g. of Florisil®. Ether was used as the eluant. Fractions shown by TLC to contain the desired methyl ester were collected and the solvent removed therefrom by evaporation *in vacuo*. Recrystallization of the solid ester from an ether-hexane solvent mixture yielded methyl β-(1-isoquinolinyl)acrylate melting at 57—9°C.; yield = 895 mg.

| | | | |
|---|---|---|---|
| Analysis Calculated: | C, 73.23; | H, 5.20; | N, 6.57 |
| Found: | C, 72.97; | H, 5.06; | N, 6.28 |

In an alternative procedure in which the intermediates were not isolated, 22 g of 1-(3,3,3-trichloro-2-hydroxypropyl)isoquinoline hydrochloride were added to a solution of 200 ml. of 50% aqueous sodium hydroxide, 100 ml. of water and 300 ml. of ethanol held at 25°C. with cooling. The reaction mixture was stirred overnight under a nitrogen atmosphere; then cooled and acidified to a pH of about 2. The volatile constituents were removed by evaporation *in vacuo*. The solid residue was triturated with methanol and filtered. The filtrate was diluted with 350 ml. of methanol and cooled. 15 ml. of thionyl chloride were thereto added in dropwise fashion. The subsequent reaction mixture was stirred at ambient temperature overnight and then diluted with water. The aqueous mixture was made basic with 14N aqueous ammonium hydroxide and the resulting alkaline solution extracted several times with ethyl acetate. The ethyl acetate extracts were separated and combined and the combined extracts washed with saturated aqeuous sodium chloride and where then dried. Evaporation of the solvent yielded 6.20 g of methyl β-(1-isoquinolinyl)-acrylate.

A reaction mixture was prepared containing 24.5 g of methyl β-(1-isoquinolinyl)acrylate, 15 g, of methyl fluorosulfonate and 400 ml. of methylenedichlororide. The reaction mixture was heated to refluxing temperature under a nitrogen atmosphere for about 16 hours whereupon it was cooled and the volatile constituents removed *in vacuo*. The residue, comprising the fluorosulfonate salt of methyl β-(2-methyl-1-isoquinolinyl)acrylate formed in the above reaction, was dissolved in about 800 ml. of methanol. 25 g. of sodium borohydride were added to this solution in small portions. After the reaction had gone to completion, the reaction mixture was diluted with water and the aqueous mixture extracted several times with ethyl acetate. The ethyl acetate extracts were separated and combined and the combined extracts washed with saturated aqueous sodium chloride and then dried. Evaporation of the ethyl acetate yielding 22.5 g. of methyl β-(2-methyl-1,2,3,4-tetrahydro-1-isoquinolinyl)acrylate formed in the above reaction.

In another run in which the isoquinolinylacrylate ester was quaternized with methyl iodide in acetonitrile, borohydride reduction yielded 1.03 g. of methyl β-(2-methyl-1,2,3,4-tetrahydro-1-isoquinolinyl)acrylate.

The hydrochloride salt was prepared by dissolving the oily free base in ether and saturating the etheral solution with gaseous hydrogen chloride. The hydrochloride salt was recrystallized from a mixture of methanol and ether; melting point = 162—4°C. with decomposition. The salt had the following elemental analysis.

| | | | | |
|---|---|---|---|---|
| Calculated: | C, 62.80; | H, 6.78; | N, 5.23; | Cl, 13.24 |
| Found: | C, 62.58; | H, 6.57; | N, 5.19; | Cl, 13.37 |

0.73 g. of methyl β-(2-methyl-1,2,3,4-tetrahydro-1-isoquinolinyl)acrylate were hydrogenated over 1 g. of Raney nickel in 98 ml. of methanol at 421·8 g/cm.$^2$ (4.1 × $10^5$Pa). The hydrogenation mixture was filtered and the volatile constituents were removed by evaporation *in vacuo*. The resulting residue was dissolved in ether and the ethereal solution filtered through florisil. The ethereal filtrate was saturated with gaseous hydrogen chloride and the resulting precipitate crystallized from a mixture of methanol and ether to yield methyl β-(2-methyl-1,2,3,4-tetrahydro-1-isoquinolinyl)propionate hydrochloride melting at 110—112°C; yield = mg.

| | | | | |
|---|---|---|---|---|
| Analysis Calculated: | C, 62.33; | H, 7.47; | N, 5.19; | Cl, 13.14 |
| Found: | C, 62.59; | H, 7.41; | N, 5.26; | Cl, 13.22 |

A solution was prepared from 1.39 grams of the above ester hydrochloride and 100 ml. of 1N aqueous hydrochloric acid. The resulting acidic solution was heated to refluxing temperature for about 25 hours. The reaction mixture was then cooled and the volatile constituents evaporated *in vacuo*. The resulting residue, comprising β-(2-methyl-1,2,3,4-tetrahydro-1-isoquinolinyl)propionic acid hydrochloride formed in the above hydrolysis, was triturated with about 250 ml. of boiling acetone. The triturate was filtered and the filtrate concentrated to about 100 ml. and then cooled. 320 mg. of β-(2-methyl-1,2,3,4-tetrahydro-1-isoquinolinyl)propionic acid hydrochloride thus prepared melted at 147—9°C. with decomposition; yield = 320 mg.

| | | | |
|---|---|---|---|
| Analysis Calculated: | C, 61.05; | H, 7.09; | N, 5.48 |
| Found: | C, 61.19; | H, 6.88; | N, 5.48 |

In a similar run, using 22.5 g. of the unsaturated ester starting material, Raney nickel reduction followed by hydrolysis of the ester group yielded 13.2 g. of the free propionic acid salt melting at 141—4°C. with decomposition. In a third run, 5.3 g. of methyl β-(2-methyl-1,2,3,4-tetrahydro-1-isoquinolinyl)propionate yielded 4.3 g. of β-(2-methyl-1,2,3,4-tetrahydro-1-isoquinolinyl)propionic acid hydrochloride melting at 144—7°C.

Four and three-tenths grams of β-(2-methyl-1,2,3,4-tetrahydro-1-isoquinolinyl)propionic acid hydrochloride were added in small portions of 80 ml. of 18M aqueous sulfuric acid plus 20 ml. of fuming sulfuric acid (about 65% $SO_3$). This mixture was heated in the range of 85—90°C. for about 2.5 hours under a nitrogen atmosphere. The mixture was then poured onto ice and the resulting aqueous solution made basic by the addition of 50% aqueous sodium hydroxide. The resulting alkaline solution was extracted several times with equal volumes of methylene dichloride. The methylene dichloride extracts were combined and the combined extracts washed with saturated aqueous sodium chloride. The combined

extracts were dried and the solvent removed therefrom by evaporation *in vacuo* to yield 2.60 g. of amorphous 1-methyl-2,3,7,8,9,9a-hexahydro-7-oxo-1H-benzo[de]quinoline formed in the above reaction.

In a second run, 2.6 g. of starting material yielded 0.37 g. of free base.

The free base was dissolved in ether and the ethereal solution saturated with gaseous hydrogen chloride to yield the corresponding hydrochloride salt. After recrystallizing from a methanol-ether solvent mixture, 1-methyl-7-oxo-2,3,7,8,9,9a-hexahydro-1H-benzo[de]quinoline hydrochloride was obtained in essentially quantitative yield, melting at about 275°C. with decomposition.

| | | | | |
|---|---|---|---|---|
| Analysis Calculated: | C, 65,68; | H, 6.78; | N, 5.89; | Cl, 14.91 |
| Found: | C, 65.48; | H, 6.54; | N, 6.08; | Cl, 14.64 |

A ring closure procedure utilizing phosphorus pentoxide and methanesulfonic acid yielded 2.0 g of the 1H-benzo[de]quinoline based upon 2.4 g. of β-(2-methyl-2,3,4-tetrahydro-1-isoquinolinyl)propionic acid hydrochloride.

A mixture of 2 g. of 1-methyl-7-oxo-2,3,7,8,9,9a-hexahydro-1H-benzo[de]quinoline and 50 g. of dimethylformamide acetal were refluxed under a nitrogen atmosphere for about 15.5 hours. The reaction mixture was cooled and the volatile constituents removed by evaporation *in vacuo*. A solution of the residue was filtered through Florisil®. Evaporation of the solvent yielded 1.9 g. of a compound showing only one major spot and no starting material by TLC. Recrystallization of this same product obtained in a similar run from an ether-hexane solvent mixture yielded 620 mg. of 1-methyl-7-oxo-8-dimethylaminomethylene-2,3,7,8,9,9a-hexahydro-1H-benzo[de]quinoline. m.p. 99—101°C;

| nmr: (δ) | H's | multiplicity | Group |
|---|---|---|---|
| 7.8—8.0 | 2 | m | aryl-H and —C=C—H |
| 7.2—7.4 | 2 | m | aryl-H |
| 3.2 | 6 | s | N—$(CH_3)_2$ (8 position) |
| 2.5 | 3 | s | N—$CH_3$ (1 position) |

| | | | |
|---|---|---|---|
| Analysis Calculated: | C, 74.97; | H, 7.86; | N, 10.93 |
| Found: | C, 74.74; | H, 7.93; | N, 10.64 |

3.2 g. of 1-methyl-7-oxo-8-dimethylaminomethylene-2,3,7,8,9,9a-hexahydro-1H-benzo[de]quinoline were dissolved in 100 ml. of methanol. Two milliliters of anhydrous hydrazine were added and the resulting mixture stirred under a nitrogen atmosphere at ambient temperature for about 24 hours. The reaction mixture was then heated to reflux under a nitrogen atmosphere for about 4 hours after which time it was cooled and the solvents evaporated *in vacuo*. The resulting residue was dissolved in chloroform and the chloroform solution chromatographed over 35 g. of florisil using chloroform containing increasing amounts (2—5%) of methanol as the eluant. Fractions shown by TLC to contain a single substance were combined. Fractions shown to contain more than one substance were also combined and rechromatographed. 2.0 g. of purified amorphous 6-methyl-4,5,6,6a,7,9-hexahydroindazolo[7,6,5-ij]isoquinoline and its tautomer, 6-methyl-4,5,6,6a,7,10-hexahydroindazolo[7,6,5-ij]isoquinoline formed in the above reaction were obtained. The product was amorphous. The hydrochloride salts of the tautomeric mixture was prepared by dissolving the amorphous free base in ethanol and adding 0.7 ml. of 12N aqueous hydrochloric acid thereto. Recrystallization of the solid hydrochloride salt from methanol yielded, essentially quantatively, crystals melting at about 260°C. with decomposition.

| | | | | |
|---|---|---|---|---|
| Analysis Calculated: | C, 64.24; | H, 6.16; | N, 16.05; | Cl, 13.54 |
| Found: | C, 64.44; | H, 6.10; | N, 16.26; | Cl, 13.58 |

The dihydrochloride salt was also prepared by using an excess of hydrochloric acid. This salt melted above 240°C. with decomposition.

Example 2

Preparation of 6-n-Propyl-4,5,6,6a,7,9-hexahydroindazolo[7,6,5-ij]isoquinoline and 6-n-Propyl-4,5,6,6a,7,10-hexahydroindazolo[7,6,5-ij]isoquinoline

Following the procedure of Example 1, 22 g. of 1-(3,3,3-trichloro-2-hydroxypropyl)isoquinoline hydrochloride were reacted with concentrated aqueous sodium hydroxide for form β-(1-isoquinolinyl)acrylic acid. Reaction of the acid with thionyl chloride formed the acid chloride. Reaction of the acid chloride with methanol yielded the corresponding methyl ester in 49% yield. Following the procedure of Example 1, the acrylate ester was hydrogenated over Raney nickel to yield methyl β-(1-isoquinolinyl)propionate. 18 g. of a yellow oil (80.5% yield) were recovered.

Next the isoquinolinyl nitrogen was quaternized with propyl iodide in acetonitrile as a solvent. The resulting quaternary salt was reduced with sodium borohydride in ethanol to yield methyl β-(2-n-propyl-1,2,3,4-tetrahydro-1-isoquinolinyl)propionate. 7.7 g. of the starting material yielded 4.2 g. of the tetrahydro derivative (44.9% yield). The corresponding hydrochloride salt was prepared in ethereal solution with gaseous HCl; M.P. = 151—3°C.

Mass spectrum; m/e = 215 (free base)

Analysis Calculated: C, 62.28; H, 5.23; N, 5.59; O, 12.76; Cl, 14.14
Found: C, 62.10; H, 5.40; N, 5.43; O, 12.93; Cl, 14.35

About 4.2 g. of methyl β-(2-n-propyl-1,2,3,4-tetrahydro-1-isoquinolinyl)propionate were stirred for about 20 hours in the presence of 200 ml. of 1N aqueous hydrochloric acid at ambient temperature under a nitrogen atmosphere. The volatile constituents were removed by evaporation and the glassy residue treated with acetone. A white solid separated comprising β-(2-n-propyl-1,2,3,4-tetrahydro-1-isoquinolinyl)propionic acid hydrochloride was obtained melting at about 208—209°C; yield = 2.49 g.

A suspension was prepared by adding 5 g. of phosphorous pentoxide to 50 g. of methanesulfonic acid (35 ml.). To this suspension were added 3.5 g. of β-(2-n-propyl-1,2,3,4-tetrahydro-1-isoquinolinyl)propionic acid hydrochloride. The reaction mixture was heated in an oil bath at a temperature in the range 85—90°C. for about one hour under a nitrogen atmosphere. The reaction mixture was then poured over ice and the consequent aqueous mixture made basic by the addition of 50% aqueous sodium hydroxide. The alkaline mixture was extracted several times with equal volumes of methylene dichloride. The organic extracts were combined and the combined extracts washed with saturated aqueous sodium chloride and then dried. Removal of the solvent *in vacuo* yielded an amorphous residue comprising 1-n-propyl-7-oxo-2,3,7,8,9,9a-hexahydro-1H-benzo[de]quinoline formed in the above reaction. The residue was dissolved in ether and the ethereal solution chromatographed over 50 g. of Florisil® using ether as the eluant. Fractions containing the desired compound (as shown by TLC) were combined. The solvent was removed from the combined fractions *in vacuo* to yield 1.35 g. (47.5%) of a brown oil. An aliquot of the oily residue was dissolved in ether and the ethereal solution saturated with gaseous hydrogen chloride. 1-n-Propyl-7-oxo-2,3,7,8,9,9a-hexahydro-1H-benzo[de]quinoline hydrochloride thus formed melted above 240°C. after recrystallization from a methanol/ether solvent mixture. Mass spectrum, m/e at 229.

Analysis Calculated: C, 67.79; H, 7.59; N, 5.27; O, 6.02; Cl, 13.34
Found: C, 67.51; H, 7.60; N, 5.19; O, 6.27; Cl, 13.53

Thin layer chromatography, (9:1 chloroform/methanol, single spot) $R_f$ = 0.80.

Following the procedure of Example 1, 1.2 g. of 1-n-propyl-7-oxo-2,3,7,8,9,9a-hexahydro-1H-benzo[de]-quinoline were dissolved in benzene and the benzene solution treated with 10 g. of tris(dimethylamino)-methane (instead of DMF acetal). The reaction was carried out and the desired product purified according to the precedure of Example 1 to yield 1.4 g. of a dark red oil (94.1% yield) comprising 1-n-propyl-7-oxo-8-dimethylaminomethylene-2,3,7,8,9,9a-hexahydro-1H-benzo[de]quinoline formed in the above reaction. Mass spectrum has m/e of 284 for the major peak.

| nmr: | (δ) | H's | multiplicity | Group |
|---|---|---|---|---|
| | 3.2 | 6 | s | $—N(C\underline{H}_3)_2$ |
| | 0.9 | 3 | t | $—CH_2CH_2—C\underline{H}_3$ |
| | 7.8 | 1 | s | $=C\underline{H}—N$ |

Following the procedure of Example 1, 1.4 g. of 1-n-propyl-7-oxo-8-dimethylaminomethylene-2,3,7,8,9,9a-hexahydro-1H-benzo[de]quinoline were dissolved in methanol and the methanolic solution treated with anhydrous hydroazine to yield an amorphous tautomeric mixture of 6-n-propyl-4,5,6,6a,7,9-hexahydroindazolo[7,6,5-ij]isoquinoline and 6-n-propyl-4,5,6,6a,7,10-hexahydroindazolo[7,6,5-ij]isoquinoline. The tautomeric mixture was isolated as the maleate salt which was prepared by adding an ethereal solution of maleic acid to an ethereal solution of the free base. The maleate salt melted at 178—80°C. with decomposition after repeated recrystallization from an ether-methanol solvent mixture. Yield = .360 g.

TLC (9:1 chloroform/methanol) $R_f$ = .58

Analysis Calculated: C, 65.03; H, 6.28; N, 11.37; O, 17.32
Found: C, 65.18; H, 6.43; N, 11.11; O, 17.06

Mass spectrum, m/e at 253 (free base).

### Example 3

Preparation of 1,2-Dimethoxy-6-benzyl-4,5,6,6a,7,9-hexahydroindazolo[7,6,5-ij]isoquinoline and 1,2-Dimethoxy-6-benzyl-4,5,6,6a,7,10-hexahydroindazolo[7,6,5-ij]isoquinoline

A solution was prepared by disolving 29.5 g. of methyl N-(3,4-dimethoxyphenyl)ethyl-4-oxo-4-aminobutanoate-prepared by the method of *Helv. Chim. Acta, 56* 759 (1973) — in 125 ml. of methylenedichloride. 31.3 g. of phosphorus pentachloride were added portionwise while the mixture was maintained at

a temperature of about 20°C. The reaction mixture was then poured over about 150 g. of ice and the consequent aqueous mixture made basic by the addition of 50% (w/v) aqueous potassium carbonate. The organic layer was separated and the alkaline aqueous phase extracted twice with 150 ml. portions of methylenedichloride. The methylenedichloride extracts were combined and dried. Evaporation of the methylenedichloride *in vacuo* yielded a residue comprising methyl β-(6,7-dimethoxy-3,4-dihydro-1-isoquinolinyl)propionate formed in the above reaction. The residue was twice dissolved in benzene and the benzene immediately evaporated therefrom leaving eventually a blue oil (weight = 27.7—30 g.). The oil was dissolved in 250 ml. of acetonitrile. 24 ml. of benzylbromide were added, thus forming a quaternary salt. The reaction mixture was heated to refluxing temperature under nitrogen for about 16 hours after which time it was cooled and the solvent removed therefrom by evaporation. The residue, comprising the benzylbromide quaternary salt of the isoquinoline was dissolved in 250 ml. of ethanol and 5 g. of sodium borohydride added thereto in portions with external cooling supplied. This reduction mixture was stirred for about one hour at ambient temperature after which time the solvent was removed *in vacuo* and the resulting residue dissolved in methylenedichloride. The methylenedichloride mixture was poured into cold 50% aqueous sodium carbonate. The organic layer was separated and the alkaline layer extracted several times with equal portions of methylenedichloride. The methylenedichloride extracts were combined and dried. Removal of the solvent therefrom *in vacuo* yielded a residue comprising methyl β-(6,7-dimethoxy-2-benzyl-1,2,3,4-tetrahydro-1-isoquinolinyl)propionate. The residue was taken up in 200 ml of 1N aqueous hydrochloric acid. The resulting solution was decolorized and filtered thorugh supercell. The resulting filtrate was evaporated to dryness e.g. *in vacuo*. The resulting residue was dissolved in acetone and the acetone solution poured into ether. A light solid formed which was separated by filtration. The filter cake was washed several times with ether. 19.6 g. (50.1%) of β-(6,7-dimethoxy-2-benzyl-1,2,3,4-tetrahydro-1-isoquinolinyl)propionic acid hydrochloride melting at 105—108°C. were obtained. This material was added to a solution of 57.5 g. of 18M sulfuric acid and 14.7 g. of oleum (60% $SO_3$). The reaction mixture was stirred for about 5 minutes at 85—90°C. and then poured into a mixture of 300 g. of ice and 600 ml. of water. The resulting aqueous mixture was made basic with 25% aqueous sodium hydroxide and the resulting alkaline phase extracted several times with equal volumes of methylenedichloride. The methylenedichloride extracts were combined and the combined extracts dried. Evaporation of the solvent *in vacuo* left an oily residue which was dissolved in ether and chromatographed over 150 g. of Florisil® using ether as the eluant. Fractions shown by TLC to contain 1-benzyl-5,6-dimethoxy-7-oxo-2,3,7,8,9,9a-hexahydro-1H-benzo[de]quinoline formed in the above reaction were combined. The column was then eluted with chloroform and an additional quantity of material was obtained; total yield = 5.2 g. (30.8% yield). An aliquot of the bezo[de]quinoline eluted with ether and shown to be one spot with an $R_f$ = .85 in a 9:1 chloroform/methanol solvent system was dissolved in ether and the ethereal solution was saturated with gaseous hydrogen chloride. The ether was decanted from the solid hydrochloride salt. The salt melted above 205°C. after recrystallization from a methanol-ether solvent mixture.

| | | | | | |
|---|---|---|---|---|---|
| Analysis Calculated: | C, 67.46; | H, 6.47; | N, 3.75; | O, 12.84; | Cl, 9.48 |
| Found: | C, 67.40; | H, 6.44; | N, 3.53; | O, 12.67; | Cl, 9.97 |

Mass spectrum, m/e at 337 (free base)

Following the procedure of Example 2, 4.5 g. of 1-benzyl-5,6-dimethoxy-7-oxo-2,3,7,8,9,9a-hexahydro-1H-benzo[de]quinoline were dissolved in 100 ml. of benzene to which was added 10 g. of tris-(dimethylamino)methane. The reaction was carried out and the product isolated as before. 1-benzyl-5,6-dimethoxy-7-oxo-8-dimethylaminoethylene-2,3,7,8,9,9a,-hexahydro-1H-benzo[de]quinoline thus prepared was purified by chromatography over 150 g. of Florisil® using chloroform containing increasing quantities (up to 2%) of methanol as the eluant. Fractions shown by TLC to contain the desired dimethylaminomethylene derivative were combined and the solvent removed therefrom by evaporation in *in vacuo*. The resulting amorphous residue was dissolved in 100 ml of anhydrous methanol to which were added 3.7 ml. of anhydrous hydrazine. The reaction was carried out and the compound isolated by the procedure of Example 1. The tautomers, 6-benzyl-1,2-dimethoxy-4,5,6,6a,7,9-hexahydroindazolo[7,6,-1j]isoquinoline and the corresponding 4,5,6,6a,7,10-hexahydro derivative were purified by chromatography over silica gel using chloroform containing increasing quantities (0.5—5%) of methanol as the eluant. Fractions shown by TLC to contain the desired indazoloisoquinoline were combined and the solvents removed therefrom *in vacuo*. The residual glassy oil was recrystalized from the ether/hexane solvent mixture to yield yellowish crystals melting at 153—156°C.

The corresponding maleate salt was prepared in essentially quantative yield by dissolving the free base in methanol and adding an ethereal solution of maleic acid. The maleate salt of 6-benzyl-1,2-dimethoxy-4,5,6,6a,7,9-hexahydroindazolo[7,6,5-ij]isoquinoline and the corresponding 4,5,6,6a,7,10-hexahydro derivative melted at 173.5—175°C.

| nmr: | (δ) | H's | multiplicity | Group |
|---|---|---|---|---|
| | 3.9 | 6 | d | —OCH$\underline{H}_3$) both |
| | 6.5 | 2 | s | —C$\underline{H}_2$—ϕ |
| | 7.5 | 1 | s | 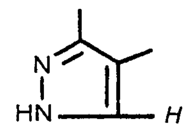 |

The maleate salt had the following analysis.
Calculated: C, 65.90; H, 5.70; N, 8.80
Found: C, 65.63; H, 5.89; N, 8.53
Mass spectrum, m/e at 361 (free base).

Example 4

Preparation of 1,2-Dimethoxy-6-methyl-4,5,6,6a,7,9-hexahydroindazolo[7,6,5-ij]isoquinoline and 1,2-Dimethoxy-6-methyl-4,5,6,6a,7,10-hexahydroindazolo[7,6,5-ij]isoquinoline

Thirty grams of methyl β-(6,7-dimethoxy-3,4-dihydro-1-isoquinolinyl)propionate prepared by the procedure of *Helv. Chim. Acta., 56,* 759 (1973) were dissolved in 125 ml. of toluene. 28.4 g. of methyl iodide were added and the mixture heated to refluxing temperature for about 5 hours. The toluene was separated from the quaternized dihydroisoquinoline by decantation and any residual toluene removed by evaporation *in vacuo*. The resulting dark blue residue was dissolved in 200 ml. of ethanol to which were added 8.2 g. of sodium cyanoborohydride portionwise. External cooling was supplied. The reaction mixture remained at ambient temperature for about oned-half hour after which time methylenedichloride was added and the resulting mixture poured over a mixture of 50 g. of ice and 50 ml. of 50% aqueous potassium carbonate. The organic layer was separated and the aqueous layer extracted three times with 100 ml portions of methylenedichloride. The methylenedichloride extracts were combined and the combined extracts dried. The solvent was removed therefrom by evaporation *in vacuo*. The resulting residue comprising methyl β-(6,7-dimethoxy-2-methyl-1,2,3,4-tetrahydro-1-isoquinolinyl)propionate formed in the above reaction was dissolved in 200 ml. of 1N aqueous hydrochloric acid. The resulting solution was decolorized with carbon and filtered through supercell. Evaporation of the filtrate to dryness yielded an oily residue. Upon addition of acetone, the residue crystalized yielding a finely divided white solid; weight = 14.5 g. β-(6.7-dimethoxy-2-methyl-1,2,3,4-tetrahydro-1-isoquinolinyl)propionic acid hydrochloride thus prepared melted 235—237°C.

Following the procedure of Example 3, β-(6,7-dimethoxy-2-methyl-1,2,3,4-tetrahydro-1-isoquinolinyl)-propionic acid hydrochloride was crystalized with fuming sulfuric acid to give 1-methyl-5,6-dimethoxy-7-oxo-2,3,7,8,9,9a-hexahydro-1H-benzo[de]quinoline. 5.3 g. of a faintly yellow transparent oil comprising the tricyclic ketone were obtained from 8.6 g. of the propionic acid derivative. This product is described by Schneider et al., *Helv. Chim. Acta, 56* 759 (1973).

Following the procedure of Example 1, 6.2 g. of 1-methyl-5,6-dimethoxy-7-oxo-2,3,7,8,9,9a-hexahydro-1H-benzo[de]quinoline were reacted with 50 ml. of DMF acetal. The reaction was carried out and the product isolated by the procedure of Example 1. Chromatography of the isolated product over Florisil® using chloroform containing increasing amounts (up to 1%) of methanol yielded 4.1 g. of a yellow solid comprising 1-methyl-5,6-dimethoxy-7-oxo-8-dimethylaminomethylene-2,3,7,8,9,9a-hexahydro-1H-benzo-[de]quinoline formed in the above reaction. m.p. 160—162°C.

| nmr: | (δ) | H's | multiplicity | Group |
|---|---|---|---|---|
| | 3.2 | 6 | s | —N—(C$\underline{H}_3$)$_2$ |
| | 2.6 | 3 | s | —N—C$\underline{H}_3$ (1-position) |
| | 6.8 | 1 | s | 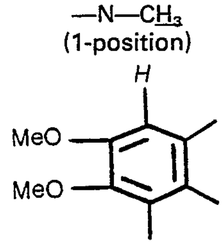 |
| | 7.8 | 1 | s | 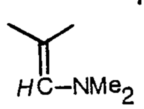 |

Treatment of the above dimethylaminomethylene derivative with anhydrous hydrazine by the procedure of Example 1 yielded 2.2 g. (from 4.1 g. of starting material of 1,2-dimethoxy-6-methyl-4,5,6,6a,7,9-hexahydroindazolo[7,6,5—ij]isoquinoline dihydrochloride and its 4,5,6,6a,7,10-hexahydro tautomer dihydrochloride, melting with decomposition at above 230°C. The dihydrochloride salts were converted to the free bases by standard procedures. The free bases were dissolved in ether to which was added a saturated solution of maleic acid. The maleate salts of 1,2-dimethoxy-6-methyl-4,5,6,6a,7,9-hexahydroindazolo[7,6,5—ij]isoquinoline and the corresponding 4,5,6,6a,7,10-hexahydro tautomer melted at about 192.5—194°C.

| nmr | (δ) | H's | multiplicity | Group |
|---|---|---|---|---|
| | 2.6 | 3 | s | N—$C\underline{H_3}$ |
| | 3.9 | 6 | d | —$OC\underline{H_3}$ (both) |
| | 6.6 | 1 | s | 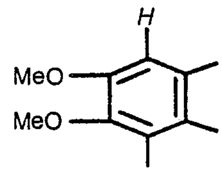 |
| | 7.5 | 1 | s | 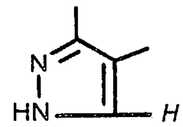 |

Analysis Calculated: C, 59.84; H, 5.78; N, 10.47; O, 23.91
Found: C, 59.65; H, 5.52; N, 10.48; O, 23.69
Mass spectrum, m/e at 285 (free base).

Example 5

Preparation of 1,2-Dimethoxy-6-n-propyl-4,5,6,6a,7,9-hexahydroindazolo[7,6,5-ij]isoquinoline and 1,2-Dimethoxy-6-n-propyl-4,5,6,6a,7,10-hexahydroindazolo[7,6,5-ij]isoquinoline

Following the procedure of Example 4, 27.7 g of methyl β-[1-(6,7-dimethoxy-3,4-dihydroisoquinolinyl)]-propionate were quaternized with n-propyl iodide. The quaternary salt was in turn reduced with sodium borohydride following the procedure of Example 4 and the methyl ester thus obtained hydrolyzed to yield β-[1-(6,7-dimethoxy-2-n-propyl-1,2,3,4-tetrahydroisoquinolinyl)]propionic acid hydrochloride melting at about 172—174°C. after recrystallization from acetone. The propionic acid hydrochloride was cyclized with oleum to yield 1-n-propyl-5,6-dimethoxy-7-oxo-2,3,7,8,9,9a-hexahydro-1H-benzo[de]quinoline. Yield = 79.7%. Treatment of the benzo[de]quinoline free base with methanolic hydrochloric acid yielded the corresponding hydrochloride salt which melted with decomposition at 206—207°C. after recrystallization from acetone.

Analysis Calculated: C, 62.67; H, 7.42; N, 4.30; O, 14.73; Cl, 10.88
Found: C, 62.41; H, 7.68; N, 4.37; O, 14.70; Cl, 11.11
Mass spectrum, m/e at 289 (free base).

Following the procedure of Example 2, 1.5 g. of the above 7-oxobenzo[de]quinoline (as the free base) were reacted with 1.3 g. of bis(dimethylamino)methoxymethane to yield 1-n-propyl-5,6-dimethoxy-7-oxo-8-dimethylaminomethylene-2,3,7,8,9,9a-hexahydro-1H-benzo[de]quinoline; yield = 1.4 g.

Thin layer chromatography (9:1 chloroform/methanol): $R_f$ = .65.

Mass spectrum, m/e at 344.

Following the procedure of Example 1, 1.4 g. of the above 7-oxo-β-dimethylaminomethylene derivative were reacted with 5 ml. of anhydrous hydrazine. A tautomeric mixture of 1,2-dimethoxy-6-n-propyl-4,5,6,6a,7,9-hexahydroindazolo[7,6,5-ij]isoquinoline and 1,2-dimethoxy-6-n-propyl-4,5,6,6a,7,10-hexahydroindazolo[7,6,5-ij]isoquinoline formed in the above reaction were purified by chromatography over Florisil® using chloroform containing increasing amounts (up to 2%) of methanol as the eluant. Fractions shown to contain the desired tautomeric mixture were combined and the solvent removed by evaporation *in vacuo*. The resulting residue was dissolved in ether to which was added a saturated ethereal solution of maleic acid, thus forming the maleate salts. 0.4 Gram of a light yellow solid were obtained which melted at 187.5—188°C. after recrystallization from an acetone-methanol solvent mixture.

Analysis Calculated: C, 61.53; H, 6.43; N, 9.78
Found: C, 61.29; H, 6.65; N, 9.46
Mass spectrum, m/e at 313 (free base).

## Example 6

Preparation of 1,2-Dimethoxy-6-ethyl-4,5,6,6a,7,9-hexahydroindazolo[7,6,5-ij]isoquinoline and 1,2-Dimethoxy-6-ethyl-4,5,6,6a,7,10-hexahydroindazolo-[7,6,5-ij]isoquinoline

When the procedure of Example 4 was repeated using 13.6 g. of methyl β-(6,7-dimethoxy-3,4-dihydro-1-isoquinolinyl)propionate, 60 ml. of toluene, 15.6 g. of ethyl iodide, 100 ml. of ethanol, 4.1 g. of sodium cyanoborohydride and 200 ml. of 1N hydrochloric acid, there was obtained, after recrystallization with acetone and filtration, 6.5 g. (41%) of a light tan solid, β-(6,7-dimethoxy-2-ethyl-1,2,3,4-tetrahydro-1-isoquinolinyl)propionic acid hydrochloride.

| | | | | |
|---|---|---|---|---|
| Analysis Calculated: | C, 58.27; | H, 7.33; | N, 4.25; | O, 19.40 |
| Found: | C, 54.56; | H, 7.15; | N, 4.13; | O, 20.85 |

Following the procedure of Example 3, 5.8 g. of β-(6,7-dimethoxy-2-ethyl-1,2,3,4-tetrahydro-1-isoquinolinyl)propionic acid hydrochloride was cyclized with fuming sulfuric acid to give 5.1 g. of 1-ethyl-5,6-dimethoxy-7-oxo-2,3,7,8,9,9a-hexahydro-1H-benzo[de]quinoline. After the product was slurried over flurosil, methanol, and filtered, the yield of 3.0 g. (64%). $R_f = 0.47$.

Following the procedure of Example 1, 3.0 g. of 1-ethyl-5,6-dimethoxy-7-oxo-2,3,7,8,9,9a-hexahydro-1H-benzo[de]quinoline were reacted with 7.2 g. of tris(dimethylamino)methane in 105 ml. of toluene. Chromatography of the isolated product over florisil using 9:1 of methylenedichloride:methanol yielded 3.5 g. (97%), as an orange yellow solid, with $R_f = 0.25$, of 1-ethyl-5,6-dimethoxy-7-oxo-8-dimethylaminomethylene-2,3,7,8,9,9a-hexahydro-1H-benzo[de]quinoline. Mass spectrum m/e 330.

| nmr | (δ) | H's | multiplicity | Group |
|---|---|---|---|---|
| | 7.6 | 1 | s | 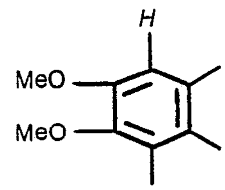 |
| | 6.6 | 1 | s | 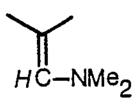 |
| | 3.9 | 6 | d | $-O-CH_3$ (both) |
| | 3.1 | 6 | s | $-N-(CH_3)_2$ |
| | 1.2 | 3 | t | $-CH_2-CH_3$ |

Reaction of the above dimethylaminomethylene derivative, 3.5 g. with 7.4 ml. anhydrous hydrazine by the procedure of Example 1 yielded 2.2 g. (69%) of 1,2-dimethoxy-6-ethyl-4,5,6,6a,7,9-hexahydroindazolo-[7,6,5,-ij]isoquinoline and its 4,5,6,6a,7,10-hexahydro tautomer.

| nmr | (δ) | H's | multiplicity | Group |
|---|---|---|---|---|
| | 7.4 | 1 | s | 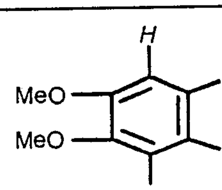 |
| | 6.5 | 1 | s | 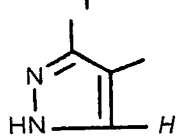 |
| | 3.9 | 6 | d | $-O-CH_3$ (both) |
| | 1.2 | 3 | t | $-CH_2-CH_3$ |

The product was dissolved in hot methanol and saturated maleic acid added. The white precipitate

which formed was filitered and washed with ether to yield 2.4 g. of the maleate salts of 1,2-dimethoxy-6-ethy-4,5,6,6a,7,9-hexahydroindazolo(7,6,5,-ij)isoquinoline and the corresponding 4,5,6,6a,7,10-hexahydro tautomer. m.p. 223°C. (decomposition). Mass spectrum m/e = 298.

| | | | |
|---|---|---|---|
| Analysis Calculated: | C, 60.71; | H, 6.07; | N, 10.11 |
| Found: | C, 60.80; | H, 5.99; | N, 9.87 |

Example 7

Preparation of 1,2-Dimethoxy-6-allyl-4,5,6,6a,7,9-hexahydroindazolo[7,6,5-ij]isoquinoline and 1,2-Dimethoxy-6-allyl-4,5,6,6a,7,10-hexahydroindazolo[7,6,5-ij]isoquinoline

3,4-Dimethoxy-1-(methoxycarbonylethylcarbonylaminoethyl)benzene, 5.0 g., was dissolved in 15 ml. of methylenedichloride. To the solution was added 5.3 g. of phosphorus pentachloride, in portions with stirring and chilling in an ice bath. Stirring was continued in the ice bath for 15 minutes, then continued at room temperature for 3 hours. The reaction mixture was poured onto ice and 30 ml. of 50% aqueous potassium carbonate. The aqueous layer was extracted three times with methylenedichloride, the organic layers combined, stripped of solvent *in vacuo*, and washed with toluene twice. The resulting green-blue residue was dissolved in 18 ml. of acetonitrile and 5.68 g. of allyl iodide was added. The reaction mixture was refluxed under nitrogen for 16 hours. The volatiles were removed *in vacuo* and the residue taken up in 17 ml. of ethanol. An orange-red precipitate formed, 20 ml. of methanol was added, and then 2.2 g. of sodium cyanoborohydride was added with stirring and chilling in an ice bath. The solid disappeared during the reaction and the stirring continued at room temperature for one hour. The solvents were removed *in vacuo*, then the residue was treated with methylenedichloride, ice and 50% aqueous potassium carbonate. The aqueous layer was extracted with methylenedichloride, dried over sodium sulfate, and evaporated to yield methyl β-(6,7-dimethoxy-2-allyl-1,2,3,4-tetrahydro-1-isoquinolinyl)propionate. Mass spectrum parent peak, m/e = 319.

| | | | |
|---|---|---|---|
| Analysis Calculated: | C, 67.69; | H, 7.89; | N, 4.39 |
| Found: | C, 67.60; | H, 7.64; | N, 4.61 |

Methyl β-(6,7-dimethoxy-2-allyl-1,2,3,4-tetrahydro-1-isoquinolinyl)propionate, 0.0169 moles, was heated with 100 ml. of 1N hydrochloric acid for 30 minutes. The mixture was filtered to leave a brown oil. The oil had water removed under vacuum to yield 6.0 g of a yellow residue. The residue was heated in acetone and cooled to form a white precipitate which was chilled in an ice bath, filtered, washed with acetone and ether to yield 3.1 g. (54%) of β-(6,7-dimethoxy-2-allyl-1,2,3,4-tetrahydro-1-isoquinolnyl)propionic acid hydrochloride. Mass spectrum major peak at 232 (free base $—CH_2CH_2CO_2H$)

| | | | |
|---|---|---|---|
| Analysis Calculated: | C, 59.73; | H, 7.08; | N, 4.10 |
| Found: | C, 60.02; | H, 6.98; | N, 4.29 |

Following the procedure of Example 3, 2.5 g. of β-(6,7-dimethoxy-2-allyl-1,2,3,4-tetrahydro-1-isoquinolinyl)propionic acid hydrochloride was cyclized with fuming sulfuric acid to give 2.9 g. (95%) of 1-allyl-5,6-dimethoxy-7-oxo-2,3,7,8,9,9a-hexahydro-1H-benzo[de]quinoline.

| nmr | (δ) | H's | multiplicity | Group |
|---|---|---|---|---|
| | 6.8 | 1 | s | 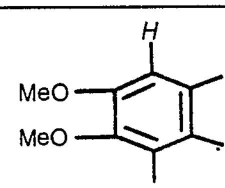 |
| | 3.9 | 6 | s | $—O—C\underline{H_3}$ (both) |

| | | | |
|---|---|---|---|
| Analysis Calculated: | C, 71.06; | H, 7.37; | N, 4.87 |
| Found: | C, 70.75; | H, 7.48; | N, 5.08 |

A small amount of the above benzo[de]quinoline free base was dissolved in ether and hydrogen chloride gas bubbled into the solution. A white precipitate formed which was filtered, washed with acetone and ether to yield 1-allyl-5,6-dimethoxy-7-oxo-2,3,7,8,9,9a-hexahydro-1H-benzo[de]quinoline hydrochloride, m.p. 186—188°C.

Following the procedure of Example 1, 1.9 g. of 1-allyl-5,6-dimethoxy-7-oxo-2,3,7,8,9,9a-hexahydro-1H-benzo[de]quinoline were reacted with 4.4 g. of tris(dimethylamino)methane in 60 ml. of toluene. Chromatography of the product over Fluorisil® using 9:1 methylenedichloride:methanol yielded 2.1 g. (92%), with $R_f = 0.54$, of 1-allyl-5,6-dimethoxy-7-oxo-8-dimethylaminoethylene-2,3,7,8,9,9a

hexahydro-1H-benzo[de]quinoline.

| nmr | (δ) | H's | multiplicity | Group |
|---|---|---|---|---|
| | 7.6 | 1 | s | 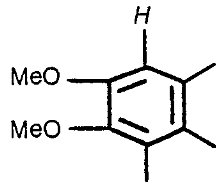 |
| | 6.6 | 1 | s | 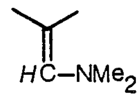 |
| | 3.8 | 6 | d | $—OC\underline{H_3}$ (both) |
| | 3.0 | 6 | s | $—N(C\underline{H_3})_2$ |

Reaction of the above dimethylaminoethylene derivative, 2.1 g. with 4.1 ml. of anhydrous hydrazine by the procedure of Example 1, yielded 360 mg. (19%) of 1,2-dimethoxy-6-allyl-4,5,6,6a,7,9-hexahydroindazolo[7,6,5-ij]isoquinoline and its 4,5,6,6a,7,10-hexahydro tautomer.

| nmr | (δ) | H's | multiplicity | Group |
|---|---|---|---|---|
| | 7.4 | 1 | s | 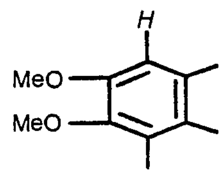 |
| | 6.4 | 1 | s | 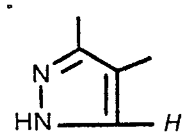 |
| | 3.8 | 6 | d | $—OC\underline{H_3}$ (both) |

The product was dissolved in ether and a small amount of hot methanol and saturated maleic acid was added. The precipitate which formed was chilled in an ice bath, filtered, and washed with ether to yield 300 mg. of the maleate salts of 1,2-dimethoxy-6-allyl-4,5,6,6a,7,9-hexahydroindazolo[7,6,5,-ij]isoquinoline and its 4,5,6,6a,7,10-hexahydro tautomer.

| | | | |
|---|---|---|---|
| Analysis Calculated: | C, 61.82; | H, 5.90; | N, 9.83 |
| Found: | C, 62.09; | H, 5.67; | N, 9.89 |

Example 8

Preparation of 1,2-Dimethoxy-6-n-propyl-9-acetyl-4,5,6,6a,7,9-hexahydroisoindolo[4,5,6-ij]isoquinoline

Potassium glycinate was formed by adding 0.43 g of glycine to a stirring solution of 0.32 g. of potassium hydroxide in 100 ml. of ethanol. To the solution were added 1.6 g. of 5,6-dimethoxy-1-n-propyl-7-oxo-8-dimethylaminomethylene-2,3,7,8,9,9a-hexahydro-1H-benzo[de]quinoline, prepared as in Example 2. The reaction mixture was refluxed three hours under nitrogen, cooled, and the solvent removed *in vacuo*. The residue was taken up in 100 ml. of acetic anhydride, refluxed 45 minutes under nitrogen, cooled and evaporated *in vacuo*. The residue is dissolved in saturated aqueous $NaHCO_3$, extracted with chloroform, the organic phase washed with saturated aqueous NaCl, dried over $Na_2SO_4$, and evaporated *in vacuo*. Chromatography, 9:1 methylenedichloride:methanol showed a spot at $R_f = 0.82$. The dark residue was dissolved in chloroform, placed on 50 g. of Florisil® and eluted with chloroform to yield, as an oil, 1.1 g. (66%) of 1,2-dimethoxy-6-n-propyl-9-acetyl-4,5,6,6a,7,9-hexahydroisoindolo[4,5,6-ij]isoquinoline. Chromatography 9:1 chloroform:methanol showed a single spot $R_f = 0.78$.

The above oil was dissolved in acetone and 0.36 g. of maleic acid in ether was added. The solvent was removed *in vacuo* and the residue crystallized from acetone/ether. A light green solid formed which was

filtered, washed with ether and dried. Chromatography 9:1 chloroform-acetone showed a single spot $R_f = 0.80$. The product, 0.25 g., was the maleate salt of 1,2-dimethoxy-6-n-propyl-9-acetyl-4,5,6,6a,7;9-hexahydroisoindolo[4,5,6-ij]isoquinoline. M.P. 85°C. (decomposition).

Mass spectrum: m/e 396 (free base) (loss of acetyl).

| | | | | |
|---|---|---|---|---|
| Analysis Calculated: | C, 63.82; | H, 6.43; | N, 5.95; | O, 23.80 |
| Found: | C, 64.10; | H, 6.28; | N, 5.91; | O, 23.97 |

| nmr (δ) | H's | multiplicity | Group |
|---|---|---|---|
| 8.2 | 1 | s | pyrrole proton |
| 7.5 | 1 | s | pyrrole proton |
| 4.0 | 6 | d | dimethoxy protons |
| 2.6 | 3 | s | N-acetyl |

The corresponding 9-hydrogen derivative of the above free base is prepared by strong basic hydrolysis using sodium ethoxide in a 1:1 molar ratio.

**Claims for the Contracting States: BE CH DE FR GB IT LI LU NL SE**

1. An 8-dimethylaminomethylene compound of the formula

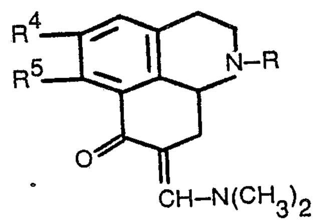

II

wherein
R is H, benzyl, $(C_1—C_3)$alkyl or allyl; and
$R^4$ and $R^5$ are independently H or $(C_1—C_3)$alkoxy.

2. A 7-oxo-hexahydrobenzo[de]quinoline of the formula

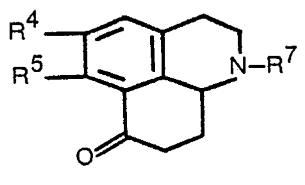

IV

wherein
$R^7$ is $(C_1—C_3)$alkyl, allyl, or benzyl;
$R^4$ and $R^5$ are independently $(C_1—C_3)$alkoxy, or H, with the limitation that when $R^4$ and $R^5$ are both $(C_1—C_3)$alkoxy, then $R^7$ can not be methyl.

3. A compound of claim 1 or claim 2 wherein $R^4$ and $R^5$ are both H and R or $R^7$ is methyl or n-propyl.

4. A compound of claim 1 or claim 2 wherein $R^4$ and $R^5$ are both methoxy and R or $R^7$ is methyl, ethyl, n-propyl, allyl or benzyl, with the proviso that $R^7$ is not methyl in a compound of claim 2.

5. A process for preparing a compound of formula II as claimed in claim 1 which comprises reacting a compound of formula

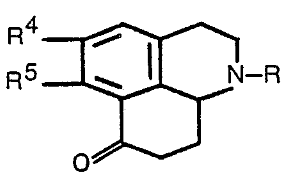

III

wherein
R, $R^4$ and $R^5$ are defined as in Claim 1, with dimethylformamide acetal, tris(dimethylamino)methane, or bis(dimethylamino)methoxymethane.

6. A process for preparing a compound of formula IV as claimed in claim 2 which comprises reacting a compound of formula

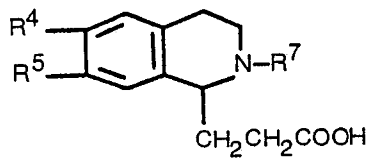

V

wherein
$R^4$, $R^5$ and $R^7$ are defined as in Claim 2 with a strong acid cyclizing agent:

**Claims for the Contracting State: AT**

1. A process for preparing a compound of the formula

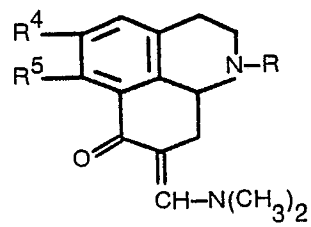

II

wherein
R is H, $(C_1—C_3)$alkyl, allyl or benzyl; and $R^4$ and $R^5$ are independently H or $(C_1—C_3)$alkoxy; which is characterized by reacting a compound of the formula

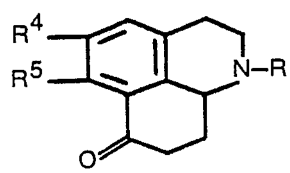

III

wherein
R, $R^4$ and $R^5$ are defined as above, with dimethylformamide acetal, tris(dimethylamino)methane, or bis-(dimethylamino)methoxymethane.

2. A process for preparing a compound of the formula

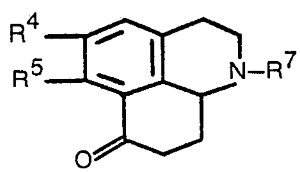

IV

wherein
$R^7$ is $(C_1—C_3)$alkyl, allyl, or benzyl;
$R^4$ and $R^5$ are independently $(C_1—C_3)$alkoxy or H, with the limitation that when $R^4$ and $R^5$ are both $(C_1—C_3)$alkoxy, then $R^7$ can not be methyl;
which is characterized by reacting a compound of the formula

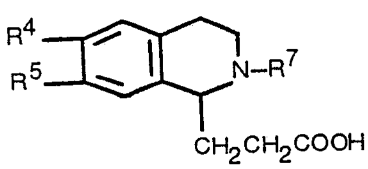

V

wherein $R^4$, $R^5$ and $R^7$ are defined as above with a strong acid cyclizing agent.

3. A process of claim 1 or claim 2 for preparing a compound of formula II or formula IV wherein $R^4$ and $R^5$ are both H and R or $R^7$ is methyl or propyl.

4. A process of claim 1 or claim 2 for preparing a compound of formula II or formula IV wherein $R^4$ and

$R^5$ are both methoxy and R or $R^7$ is methyl, ethyl, n-propyl, allyl or benzyl, with the proviso that $R^7$ is not methyl in the process of claim 2.

**Patentansprüche für die Vertragsstaaten: BE CH DE FR GB IT LI LU NL SE**

1. 8-Dimethylaminomethylenverbindung der Formel

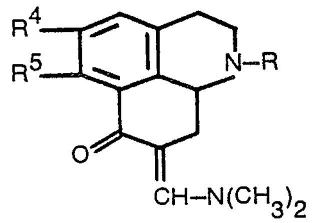

II

worin
R für H, Benzyl, ($C_1$—$C_3$)-Alkyl oder Allyl steht und
$R^4$ sowie $R^5$ unabhängig H oder ($C_1$—$C_3$)-Alkoxy sind.

2. 7-Oxohexahydrobenzo[de]chinoline der Formel

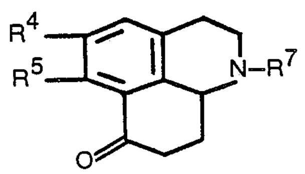

IV

worin
$R^7$ für ($C_1$—$C_3$)-Alkyl, Allyl oder Benzyl steht,
$R^4$ sowie $R^5$ unabhängig ($C_1$—$C_3$)-Alkoxy oder H sind, mit der Maßgabe, daß, falls $R^4$ und $R^5$ jeweils ($C_1$—$C_3$)-Alkoxy sind, der Substituent $R^7$ dann nicht Methyl sein kann.

3. Verbindung nach Anspruch 1 oder 2, dadurch gekennzeichnet, daß $R^4$ und $R^5$ jeweils H sind und R oder $R^7$ Methyl oder n-Propylist.

4. Verbindung nach Anspruch 1 oder 2, dadurch gekennzeichnet, daß $R^4$ und $R^5$ jeweils Methoxy sind und R oder $R^7$ Methyl, Ethyl, n-Propyl, Allyl oder Benzyl ist, mit der Maßgabe, daß $R^7$ bei einer Verbindung nach Anspruch 2 nicht Methyl ist.

5. Verfahren zur Herstellung einer Verbindung der Formel (II) gemäß Anspruch 1, dadurch gekennzeichnet, daß man eine Verbindung der Formel

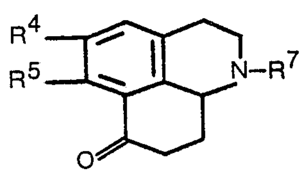

III

worin
R, $R^4$ und $R^5$ wie im Anspruch 1 definiert sind, mit Dimethylformamidacetal, Tris(dimethylamino)methan oder Bis(dimethylamino)methoxymethan umsetzt.

6. Verfahren zur Herstellung einer Verbindung der Formel (IV) gemäß Anspruch 2, dadurch gekennzeichnet, daß man eine Verbindung der Formel

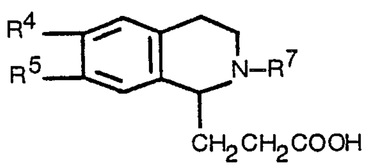

V

worin
$R^4$, $R^5$ und $R^7$ wie im Anspruch 2 definiert sind, mit einem stark sauren Cyclisierungsmittel umsetzt.

**Patentansprüche für den Vertragsstaat: AT**

1. Verfahren zur Herstellung einer Verbindung der Formel

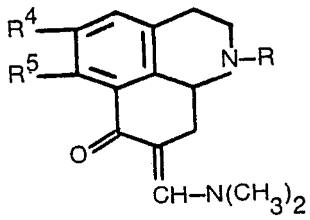

II

worin

R für H, Benzyl, ($C_1$—$C_3$)-Alkyl oder Allyl steht und $R^4$ sowie $R^5$ unabhängig H oder ($C_1$—$C_3$)-Alkoxy sind, dadurch gekennzeichnet, daß man eine Verbindung der Formel

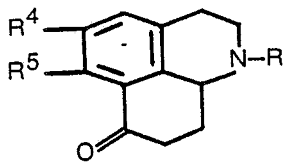

III

worin

R, $R^4$ und $R^5$ wie oben definiert sind, mit Dimethylformamidacetal, Tris(dimethylamino)methan oder Bis(dimethylamino)methoxymethan umsetzt.

2. Verfahren zur Herstellung einer Verbindung der Formel

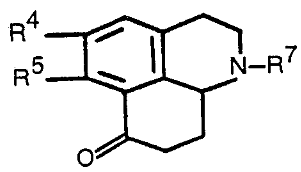

IV

worin

$R^7$ für ($C_1$—$C_3$)-Alkyl, Allyl oder Benzyl steht,

$R^4$ sowie $R^5$ unabhängig ($C_1$—$C_3$)-Alkoxy oder H sind, mit der Maßgabe, daß, falls $R^4$ und $R^5$ jeweils ($C_1$—$C_3$)-Alkoxy sind, der Substituent $R^7$ dann nicht Methyl sein kann,

dadurch gekennzeichnet, daß man eine Verbindung der Formel

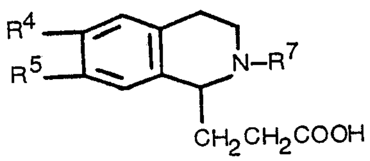

V

worin

$R^4$, $R^5$ und $R^7$ wie oben definiert sind, mit einem stark sauren Cyclisierungsmittel umsetzt.

3. Verfahren nach Anspruch 1 oder 2, dadurch gekennzeichnet, daß man eine Verbindung der Formel (III) oder (IV) herstellt, worin $R^4$ und $R^5$ jeweils H sind und R oder $R^7$ Methyl oder n-Propyl ist.

4. Verfahren nach Anspruch 1 oder 2, dadurch gekennzeichnet, daß man eine Verbindung der Formel (II) oder (IV) herstellt, worin $R^4$ und $R^5$ jeweils Methoxy sind und R oder $R^7$ Methyl, Ethyl, n-Propyl, Allyl oder Benzyl ist, mit der Maßgabe, daß $R^7$ bei einer Verbindung nach Anspruch 2 nicht Methyl ist.

**Revendications pour les Etats Contractants: BE CH DE FR GB IT LI LU NL SE**

1. Composé de 8-diméthylaminométhylène de formule:

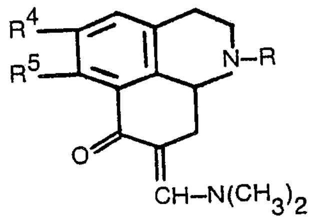

II

dans laquelle
R représente H, un groupe benzyle, un groupe alkyle en $C_1$—$C_3$ ou un groupe allyle; et
$R^4$ et $R^5$ représentent chacun indépendamment l'un de l'autre H ou un groupe alcoxy en $C_1$—$C_3$.

2. 7-oxo-hexahydrobenzo[de]quinoléine de formule:

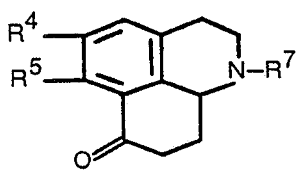

IV

dans laquelle
$R^7$ représente un groupe alkyle en $C_1$—$C_3$, un groupe allyle ou un groupe benzyle;
$R^4$ et $R^5$ représentent chacun indépendamment l'un de l'autre un groupe alcoxy en $C_1$—$C_3$, ou H, avec cette réserve que, lorsque $R^4$ et $R^5$ représentent tous deux un groupe alcoxy en $C_1$—$C_3$, $R^7$ ne peut être un groupe méthyle.

3. Composé selon la revendication 1 ou 2, dans lequel $R^4$ et $R^5$ représentent tous deux H, tandis que R ou $R^7$ représente un groupe méthyle ou un groupe n-propyle.

4. Composé selon la revendication 1 ou 2, caractérisé en ce que $R^4$ et $R^5$ représentent tous deux un groupe méthoxy, tandis que R ou $R^7$ représente un groupe méthyle, un groupe éthyle, un groupe n-propyle, un groupe allyle ou un groupe benzyle, avec cette réserve que $R^7$ ne représente pas un groupe méthyle dans un composé selon la revendication 2.

5. Procédé de préparation d'un composé de formule II selon la revendication 1, caractérisé en ce qu'il consiste à faire réagir un composé de formule:

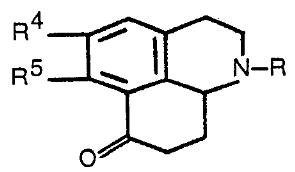

III

dans laquelle
R, $R^4$ et $R^5$ ont les significations définies dans la revendication 1, avec du diméthylformamido-acétal, du tris-(diméthylamino)méthane ou du bis-(diméthylamino)méthoxyméthane.

6. Procédé de préparation d'un composé de formule IV selon la revendication 2, caractérisé en ce qu'il consiste à faire réagir un composé de formule:

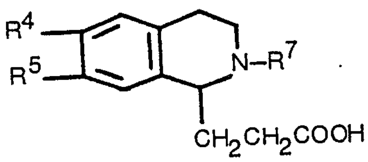

V

dans laquelle $R^4$, $R^5$ et $R^7$ ont les significations définies dans la revendication 2, avec un agent cyclisant acide fort.

**Revendications pour l'Etat Contractant: AT**

1. Procédé de préparation d'un composé de formule:

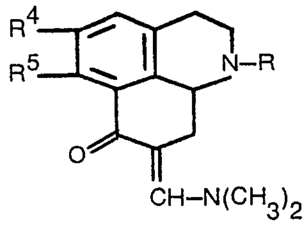

II

dans laquelle

R représente H, un groupe alkyle en $C_1$—$C_3$, un groupe allyle ou un groupe benzyle; et

$R^4$ et $R^5$ représentent chacun indépendamment l'un de l'autre H ou un groupe alcoxy en $C_1$—$C_3$; caractérisé en ce qu'il consiste à faire réagir un composé de formule:

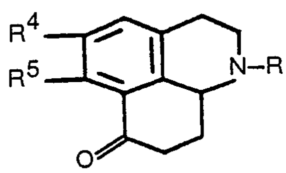

III

dans laquelle

R, $R^4$ et $R^5$ ont les significations définies ci-dessus, avec du diméthylformamido-acétal, du tris-(diméthylamino)méthane ou du bis-(diméthylamino)méthoxyméthane.

2. Procédé de préparation d'un composé répondant à la formule:

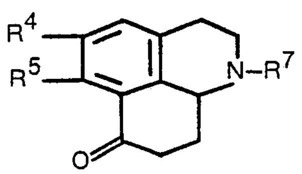

IV

dans laquelle

$R^7$ représente un groupe alkyle en $C_1$—$C_3$, un groupe allyle ou un groupe benzyle;

$R^4$ et $R^5$ représentent chacun indépendamment l'un de l'autre un groupe alcoxy en $C_1$—$C_3$ ou H, avec cette réserve que, lorsque $R^4$ et $R^5$ représentent tous deux un groupe alcoxy en $C_1$—$C_3$, $R^7$ ne peut être un groupe méthyle;

caractérisé en ce qu'il consiste à faire réagir un composé de formule:

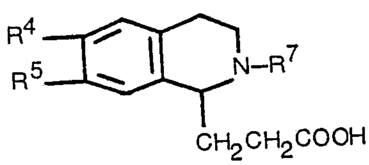

V

dans laquelle

$R^4$, $R^5$ et $R^7$ ont les significations définies ci-dessus, avec un agent cyclisant acide fort.

3. Procédé selon la revendication 1 ou 2 en vue de préparer un composé de formule II ou de formule IV où $R^4$ et $R^5$ représentent chacun H, tandis que R ou $R^7$ représente un groupe méthyle ou un groupe propyle.

4. Procédé selon la revendication 1 ou 2 en vue de préparer un composé de formule II ou de formule IV dans laquelle $R^4$ et $R^5$ représentent tous deux un groupe méthoxy, tandis que R ou $R^7$ représente un groupe méthyle, un groupe éthyle, un groupe n-propyle, un groupe allyle ou un groupe benzyle, avec cette réserve que $R^7$ ne représente pas un groupe méthyle dans le procédé de la revendication 2.